# FASCICULE DE BREVET EUROPEEN

(11) **EP 2 291 537 B1**
(45) Date de publication et mention de la délivrance du brevet: **12.10.2016**
(21) Numéro de dépôt: 09784426.0
(22) Date de dépôt: 23.06.2009
(51) Int. Cl.: C12Q 1/14, C12Q 1/04

(54) **MILIEU DE CULTURE COMPORTANT UN COMPOSE INHIBITEUR OU RETARDATEUR DE GERMINATION DE SPORES**
KULTURMEDIUM MIT EINER VERBINDUNG ZUR HEMMUNG ODER VERZÖGERUNG DER SPORENKEIMUNG
CULTURE MEDIUM CONTAINING A COMPOUND INHIBITING OR DELAYING SPORE GERMINATION

(30) Priorité: 26.06.2008 FR 0854275
(43) Date de publication de la demande: 09.03.2011
(73) Titulaire: Biomérieux, 69280 Marcy L'etoile (FR)
(72) Inventeur: COSTA, Aurélien, F-38000 Grenoble (FR)
(74) Mandataire: Sprugnoli, Claude Louis Victor
(86) Numéro de dépôt international: PCT/FR2009/051201
(87) Numéro de publication internationale: WO 2010/004164

(56) Documents cités:
- EP-A- 1 873 256
- EP-B- 1 390 524
- US-A- 6 063 590
- YASUDA Y. ET AL.: "Regulation of L-alanine germination of Bacillus subtilis spores by alanine racemase" AMINO ACIDS, vol. 4, no. 1-2, février 1993 (1993-02), pages 89-99, XP002519083

## Description

Le domaine de l'invention est celui du contrôle microbiologique clinique ou industriel. Plus particulièrement, la présente invention concerne essentiellement un milieu de culture de microorganismes cibles, susceptibles d'être présent dans un échantillon, plus particulièrement dans un échantillon alimentaire ou environnemental, ledit milieu de culture comportant au moins un composé inhibiteur ou retardateur de la germination de spores.

La présente invention sera particulièrement décrite ci-après, en référence à des produits déshydratés, sans pour autant que cela constitue une limitation à l'invention.

La qualité et la sécurité sont les soucis majeurs de l'industrie agroalimentaire, au niveau mondial. Dans les matières premières et les produits finis, il doit être possible de détecter, identifier et quantifier la flore microbienne et de garantir ainsi la valeur des produits, de leur fabrication jusqu'à leur consommation. On analyse ainsi les indicateurs-qualité du produit, reflet d'un taux de contamination global par une flore d'altération à laquelle appartiennent les entérobactéries comme *Escherichia coli,* les staphylocoques à coagulase positive, les *Pseudomonas*, les *Bacillus,* les levures, les moisissures etc. En particulier, la recherche des staphylocoques à coagulase positive dans les poudres de lait est devenue aujourd'hui incontournable.

Parmi les staphylocoques à coagulase positive, *Staphylococcats aureus* est l'espèce la plus pathogène du genre *Staphylococcus*, bien qu'étant commensale chez l'homme. En effet, elle peut être responsable d'intoxications alimentaires, d'infections localisées suppurées, et dans certains cas extrêmes, de septicémies chez des sujets ayant subi une greffe ou porteur de prothèse cardiaque.

La difficulté de la mise au point d'un milieu de culture permettant la numération spécifique des staphylocoques à coagulase positive réside :
- non seulement dans la nécessité que ce milieu de culture présente une bonne sensibilité en permettant la détection d'une contamination très faible en staphylocoques à coagulase positive (de l'ordre de 10 Unités Formant Colonie (UFC)/g) présents dans ledit milieu, mais aussi
- dans la nécessité de pouvoir inhiber des taux parfois très élevés d'autres germes, et tout particulièrement de germes tels que ceux de *Bacillus* qui sont phénotypiquement proches des staphylocoques à coagulase positive.

En effet, en ce qui concerne précisément les germes de *Bacillus,* la différenciation des staphylocoques à coagulase positive par rapport aux *Bacillus* par des agents sélectifs s'avère particulièrement délicate; la plupart des inhibiteurs efficaces sur l'une de ces bactéries affectant généralement aussi l'autre bactérie. Ainsi, il a été observé que le milieu de culture traditionnel du type Baird-Parker-RPF (plasma de lapin + fibrinogène bovin) ne permettait pas une inhibition efficace et systématique de *Bacillus.*

Par ailleurs, dans le domaine agroalimentaire, les industriels ont souvent recours à des procédés de fabrication de produits par déshydratation dont les conditions permettent généralement l'élimination de la plupart des germes, notamment sous leur forme végétative. Or, les bactéries du genre *Bacillus,* par leur capacité à former des spores, sont capables de résister à des conditions extrêmes telles que celles mises en oeuvre au cours de la déshydratation. On les retrouve alors dans les produits déshydratés. Ainsi, il a été observé que le taux de contamination en *Bacillus* de laits en poudre pouvait être important.

De plus, la plupart des matières premières nécessaires à la réalisation de milieux de culture se présentent, elles-aussi, fréquemment sous forme déshydratée (poudre).

Ces matières premières sèches sont également régulièrement contaminées par ces germes de *Bacillus* qui, tel qu'explicité *supra*, résistent aux conditions de déshydratation.

Il s'ensuit qu'il arrive fréquemment d'observer des résultats faux-positifs dans le contrôle microbiologique des produits déshydratés tels que les poudres de lait, dus au fait que des bactéries du genre *Bacillus* sont analysées, à tort, comme étant des staphylocoques à coagulase positive.

A contrario, le risque de faux-négatifs est tout aussi important dans la mesure où la quantité souvent importante de *Bacillus,* et toujours bien supérieure à celle des staphylocoques à coagulase positive, masque souvent la présence de ces derniers.

On comprend alors aisément qu'une quantité importante de *Bacillus* s'avère un obstacle majeur à la mise au point d'un milieu de culture destiné à l'identification, voire au dénombrement de bactéries cibles telles que des staphylocoques à coagulase positive et peut conduire à une mauvaise estimation de la présence de ces germes présents dans un échantillon d'analyses tel qu'un échantillon alimentaire. L'échantillon est défini comme une petite partie ou petite quantité isolée d'une entité pour analyse
EP-B-1 390 524 décrit des milieux de détection spécifique de *Staphylococcus aureuset* des procédés d'identification et/ou de dénombrement utilisant de tels milieux. La spécificité de la détection est augmentée par l'ajout dans le milieu d'un mélange d'inhibiteurs de la croissance de microorganismes non Staphylocoques susceptibles d'être présents dans le milieu.

Bien que différentes méthodes de développement d'un milieu de culture en vue du dénombrement spécifique des staphylocoques à coagulase positive aient été testées, elles n'ont pas abouti au succès escompté, quant à la limitation de l'impact des *Bacillus.*

Ainsi, des agents sélectifs de type antibiotiques ne permettent pas de combiner une bonne sélectivité vis-à-vis des germes de *Bacillus* avec le maintien d'une bonne sensibilité vis-à-vis des germes de staphylocoques à coagulase positive.

Deux publications datant de plusieurs dizaines d'années ont fait état de l'action de la D-alanine sur la germination des spores.

Ainsi, il est connu de la publication de HALMANN M, KEYNAN A., 1962, « Stages in germination of spores of Bacillus Licheniformis », J. Bacteriol. 84 :1187-1193 que la L-alanine déhydrogénase est nécessaire à la première étape biochimique de germination des spores de *Bacillus Licheniformis* et que cette première étape peut être inhibée par la D-alanine, divers sels, le pyruvate d'éthyle, et l'octanol.

Il est aussi mentionné dans la publication de YASUSA-YASAKI Yoko et al., 1978, « Inhibition of Bacillus subtilis Spore Germination by Various Hydrophobic Compounds : Demonstration of Hydrophobic Character of L-alanine Receptor Site », Journal of Bacteriology, Vol.136, n°2, p 484-490 que les inhibiteurs de la L-alanine à l'origine de l'initiation de la sporulation sont :
- l'octanol sur *Bacillus licheniformis* et *Bacillus cereus*,
- l'alcool phényléthylique sur le *Bacillus megaterium*,
- la 8-hydroxyquinoline sur le *Bacillus megaterium,* le *Bacillus cereus* et le *Bacillus subtilis*,
- l'éthanol et le dicyclohexylcarbodiimide sur le *Bacillus cereus*.

De plus, Yasuda Y. et al., 1993, "Regulation of L-alanine germination of Bacillus sobtilisspores by alanine racemase", Amino Acids, vol. 4, no. 1-2, p. 89-99, décrit l'étude de la régulation par l'alanine racémase de la germination des spores de *Bacillus sobtilis*initiée par la L-alanine et divulgue que la D-alanine et la diphénylamine sont des inhibiteurs de la germination de spores de *Bacillus subtilis*.

Les études publiées par la suite se sont essentiellement focalisées sur la caractérisation du mécanisme de germination des formes sporulées des germes, dont le genre *Bacillus* est un exemple.

Lesdites études ont précisément décrit les différentes étapes qui interviennent dans le processus de germination.

Cette connaissance du mécanisme de germination a, jusqu'à présent, été essentiellement utilisée pour favoriser la germination des spores de microorganismes, en particulier de *Bacillus,* avec plusieurs objectifs :
- Accélérer la germination et ainsi améliorer la croissance/détection dans des milieux de culture
- Favoriser la germination pour éliminer les germes sous leur forme végétative, qui sont alors beaucoup plus sensibles aux agents inhibiteurs présents sous la forme d'antibiotiques dans les milieux de culture ou sous la forme de détergents ou désinfectants dans certains procédés de nettoyages industriels, d'autres procédés étant, quant à eux, basés sur des traitements à hautes températures.

La présente invention se propose de remédier aux inconvénients susmentionnés, en apportant une solution à contre-courant de celles déjà identifiées et qui consiste non pas à favoriser la germination des spores, mais au contraire à la bloquer, ou à tout le moins, la retarder.

Elle représente par ailleurs une alternative tout à fait intéressante à l'usage d'antibiotiques, dont les spectres d'action ne sont généralement pas compatibles avec le besoin de différenciation entre les microorganismes cibles tels que les staphylocoques à coagulase et des microorganismes, tels que *Bacillus,* susceptibles d'interférer la détection, l'identification voire le dénombrement desdits microorganismes cibles.

La présente invention possède en outre l'avantage de ne pas modifier les qualités nutritives du milieu de culture, ce qui permet d'éviter les problèmes de spécificité sus-évoqués et de rendre ainsi le milieu de culture développé plus particulièrement aptes à l'analyse des produits tels que les produits déshydratés, dans lesquels le taux de *Bacillus* est important.

C'est pourquoi, l'un des objectifs essentiels de la présente invention a consisté à mettre en oeuvre un milieu de culture de microorganismes cibles, en particulier de bactéries, qui soit en mesure de bloquer, à tout le moins retarder, la germination de spores éventuellement contenues dans l'échantillon à analyser, sans que cela ait d'incidence sur la croissance desdits microorganismes cibles.

Un premier objet de l'invention consiste ainsi en un milieu de culture et de détection de staphylocoques à coagulase positive, comprenant :
- au moins un substrat spécifique, naturel ou synthétique, permettant la détection d'au moins une activité métabolique desdits staphylocoques à coagulase positive, dont le produit de dégradation fait varier le pH, un indicateur de pH permettant de révéler la consommation dudit substrat,
- au moins un composé inhibiteur ou retardateur de la germination de spores de *Bacillus* susceptible d'interférer avec la culture et la détection desdits staphylocoques à coagulase positive, ledit composé inhibiteur ou retardateur de la germination de spores étant choisi dans le groupe comprenant la D-alanine, la diphénylamine, les alcools de formule CₙH₂ₙ₊₂OH où n varie de 6 à 12, de préférence l'octanol, le nonanol, le décanol, les inhibiteurs d'enzymes de type trypsine et/ou leurs mélanges.

De façon préférentielle, les staphylocoques à coagulase positive sont *Staphylococcats aureus.*

Au sens de la présente invention, le substrat est un substrat métabolique, tel qu'une source de carbone ou d'azote, dont le produit de dégradation fait varier le pH, variation détectable gràce à un indicateur de pH. L'indicateur de pH est une substance chimique dont la couleur varie en fonction de modifications du pH associées à la croissance microbienne. On citera comme exemples de chromophores le rouge neutre, le bleu d'aniline, le bleu de bromocresol. Dans un second mode de réalisation, l'indicateur de pH est un fluorophore (par exemple la 4-méthylumbelliferone, les dérivés de l'aminocoumarine ou les dérivés de la résorufine).

Le substrat peut également être choisi parmi tout substrat pouvant être hydrolysé en un produit qui permet la détection, directe ou indirecte, d'une activité enzymatique spécifique du microorganisme recherché. Dans le cas de la détection directe, le substrat comprend une première partie spécifique de l'activité enzymatique et un deuxième partie faisant office de marqueur, qui peut être chromogène ou fluorescente.

Le milieu de culture, objet de l'invention, peut être sous forme de poudre, sous forme de gel ou sous forme liquide, prêt à l'emploi, c'est-à-dire prêt pour l'ensemencement en tube, flacon, ou sur boite de Pétri. Dans le cas d'un milieu gélifié, l'agar est l'agent gélifiant traditionnel en microbiologie pour la culture des microorganismes, mais il est possible d'utiliser de la gélatine ou de l'agarose. Enfin, le milieu peut être conditionné dans une bouteille, une cartouche ou une carte spécifiques d'un appareil automatisé de bactériologie (on citera à titre d'exemple la carte Vitek®, la carte Tempo® et la bouteille BacT/ALERT®, commercialisées par la demanderesse). Dans ce cas, le composé retardateur ou inhibiteur peut se présenter sous forme d'une solution indépendante destinée à être ajoutée au milieu de culture avant son emploi.

Parmi les échantillons d'origine alimentaire, on peut citer de façon non exhaustive un échantillon de produits déshydratés tels que les poudres de lait, les céréales, les soupes en poudres, les préparations de gâteaux. C'est le cas également des pâtisseries. Un échantillon alimentaire peut enfin être issu d'une alimentation destinée aux animaux, telle que notamment que des farines animales.

On mentionnera aussi les échantillons liés à l'environnement tels les prélèvements de surface, d'eau, d'air.

Selon un mode de réalisation préférentiel, la concentration du composé inhibiteur ou retardateur de la germination de spores, dans ledit milieu, est comprise entre 0,01 et 1 mol/L, préférentiellement 0,05 et 0,1 mol/L.

Le indicateur de pH permettant de révéler la consommation du substrat, est préférentiellement un chromophore ou un fluorophore.

Traditionnellement, le substrat peut comprendre une première partie spécifique de l'activité métabolique à révéler et une seconde partie faisant office de marqueur fluorescent.

Alternativement, le substrat peut comprendre une première partie spécifique de l'activité métabolique à révéler et une seconde partie faisant office de marqueur chromogène.

Un deuxième objet de la présente invention, concerne un procédé de détection de microorganismes cibles susceptibles d'être présents dans un échantillon, comprenant les étapes consistant à :
a) Mettre en contact ledit échantillon avec un milieu de culture selon l'invention,
b) Incuber ledit milieu de culture dans des conditions aptes à la multiplication cellulaire de microorganismes,
c) Détecter la présence desdits microorganismes cibles.

L'étape c) du procédé selon l'invention peut consister à mesurer la variation du niveau de fluorescence dans le milieu de culture à l'aide d'un lecteur de fluorescence, cette variation correspondant par exemple à une variation du pH dans ledit milieu de culture.

Alternativement, l'étape c) peut consister à identifier les colonies. Dans ce cas, le milieu de culture selon l'invention se présente typiquement sous forme solide, en particulier sous forme de milieu gélosé en boites de Pétri. La germination des spores étant retardée, les bactéries d'intérêt poussent en priorité et préalablement sur le milieu de culture, en comparaison des autres bactéries. Ainsi, dans le cas d'une lecture précoce des boites de Pétri (16 heures après l'ensemencement par exemple), seules les colonies des bactéries d'intérêt peuvent être visibles. Dans le cas d'une lecture plus tardive (24 heures après l'ensemencement par exemple), celle-ci consiste à différencier les colonies de grandes tailles, des colonies de petites tailles. Les colonies de bactéries obtenues par germination des spores sont de plus petite taille que celles des bactéries cibles qui elles se sont développer normalement. Il est alors aisé de faire la différence entre les bactéries cibles et les autres bactéries.

Selon un mode particulier de réalisation, le procédé selon l'invention peut comporter une étape supplémentaire de dénombrement de microorganismes cibles. Une telle étape de dénombrement est de préférence réalisée selon la méthode du Nombre le Plus Probable (NPP). Cette méthode est explicitée dans le brevet EP 1 105 457 au nom de la Demanderesse.

Un autre objet de la présente invention concerne l'utilisation d'au moins un composé retardateur ou inhibiteur de la germination de spores de microorganismes pour la préparation du milieu de culture de l'invention.

Un autre objet de la présente invention concerne l'utilisation de la D-alanine pour la préparation du milieu de culture de l'invention destiné à l'identification des staphylocoques à coagulase positive tel que *Staphylococcats aureus.*

Un dernier objet de l'invention concerne l'utilisation du milieu de culture de l'invention pour la détection, l'identification, voire le dénombrement des staphylocoques à coagulase positive.

La réalisation du milieu de culture selon l'invention présente l'avantage d'être totalement compatible avec les conditions traditionnelles des procédés de fabrication des milieux de culture, étant donné que les composés inhibiteur ou retardateur de la germination de spores sont généralement stables dans une plage importante de valeurs de température, et ce contrairement aux agents antibiotiques. Par ailleurs, ces composé sont souvent d'un coût de reviens réduit.

Les buts et avantages de la présente invention seront mieux compris à la lumière de l'exemple qui suit, en lien avec le dessin, dans lequel :

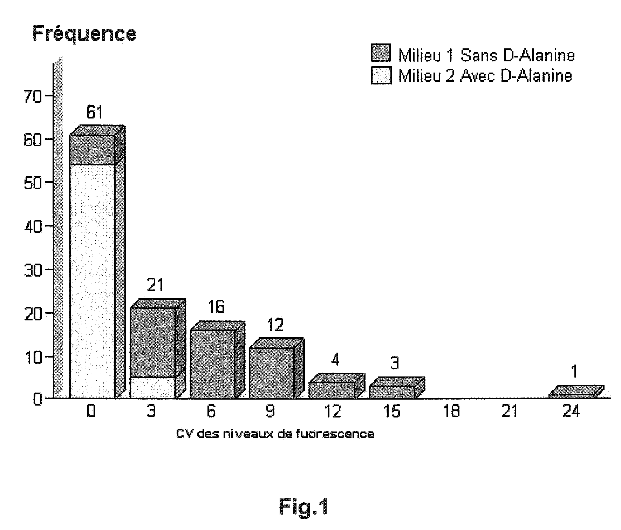

La figure 1 est un graphique en histogrammes montrant la répartition des CV relatifs aux niveaux de fluorescence obtenus après incubation avec des échantillons testés sur milieu de culture avec et sans D-alanine.

### Exemple :

L'exemple décrit ci-après illustre l'effet inhibiteur de la D-alanine sur la sporulation de germes de type *Bacillus* (tels que *Bacillus licheniformis*, *Bacillus thuringiensis*/*cereus*).

Pour ce faire, 59 échantillons de produits de poudres de lait, susceptibles de contenir des germes de *Bacillus,* non contaminés en outre par *Staphylococcus aureus,* ont été testés.

Pour étudier l'effet inhibiteur de la D-alanine, deux milieux de culture ont été testés:
- Milieu 1 : Milieu de culture sans D-Alanine,
- Milieu 2 : Milieu de culture en présence de D-Alanine à une concentration finale dans le milieu comprise entre 0,05 et 0,1 mol/L (concentration finale dans le milieu).

La composition dudit milieu de culture est la suivante (en g/L, concentration finale dans le milieu avec ou sans D-Alanine) :

| | |
|---|---|
| Peptones animales (bovines et porcines) et végétales | 12,5 |
| Sucres et suppléments de croissance | 11 |
| Système Tampon | 10 |
| Agents sélectifs (anbtibiotiques, sels) | 10,25 |
| Indicateur de pH fluorescent | 0,06 |
| Antimousse | 0,4 |

Les échantillons ont été préparés conformément à la norme ISO 8261 : 2001 (concernant le lait et les produits laitiers et relative aux « lignes directrices générales pour la préparation des échantillons pour essai, de la suspension mère et des dilutions décimales en vue de l'examen microbiologique ») selon les étapes suivantes :
a) pesée de 10 grammes de l'échantillon et placement dans un sac Stomacher®,
b) ajout de 90 mL de diluant de sel de tryptone préalablement réchauffé à 45°C pour obtenir une dilution initiale 1/10 de l'échantillon,
c) broyage à l'aide d'un appareil Stomacher® durant une minute,
d) placement et mise sous agitation du sac Stomacher® pendant 5 minutes à 45°C dans un bain Marie,
e) prélèvement de 1 mL de l'échantillon (dilué au dixième) dans 3 mL de milieu culture pour obtenir une dilution 1/40,
f) incubation pendant une durée comprise entre 24 et 27 heures à une température de 37°C,
g) lecture du milieu de culture par fluorescence et détermination du taux de contamination de l'échantillon par des germes par la technique du nombre le plus probable.

La lecture consiste en une mesure de la fluorescence du milieu de culture obtenu après incubation. Une variation importante des valeurs de fluorescence témoigne d'une acidification du milieu, elle-même résultant de la croissance d'un ou plusieurs germes.

Les CV de fluorescence ont été calculés à partir des échantillons testés (non contaminés par *Staphylococcus aureus*).

Plus le CV est élevé, plus l'acidification du milieu est importante. Ce qui témoigne d'une croissance des germes présents dans le milieu de culture. Inversement, moins le CV est élevé, moins l'acidification est importante, ce qui reflète une croissance moindre des germes. La spécificité du milieu est donc meilleure et le risque de faux positifs est donc moindre (résultat faux positif lié à l'acidification du milieu par une bactérie d'une autre germe que *Staphylococcus aureus*).

Les échantillons testés dans cet exemple n'étant pas contaminés par *Staphylococcats aureus,* les acidifications observées sont liées à la croissance non spécifique de différents germes autres que *Staphylococcus* aureus identifiés comme appartenant au genre *Bacillus* (*Bacillus licheniformis*, *Bacillus thuringiensis*/*cereus*).

Les profils obtenus et présentés sous forme d'histogrammes, tels que représentés sur la figure 1, montrent une différence importante dans la répartition des CV entre les deux milieux de culture :

Les CV obtenus avec la milieu 1 (sans D-alanine) sont répartis sur des valeurs comprises entre 0 et 24 tandis que l'ensemble des valeurs obtenues avec le milieu 2 (avec D-alanine) sont inférieures à 6 et dans leur grande majorité inférieures à 3 (très légère acidification).

L'usage de D-Alanine dans le milieu de culture permet de diminuer fortement les valeurs de CV obtenues après incubation du milieu. Les CV étant dans le cas étudié liés à des acidifications non spécifiques, l'ajout de D-Alanine dans le milieu lui confère une meilleure spécificité vis à vis des Bacillus présents sur ce type d'échantillon sous forme sporulée.

Le tableau 1 suivant récapitule les numérations obtenues avec ou sans D-alanine dans le milieu de culture.

**Tableau 1**

| **Numération NPP** | **D-Alanine testée** | | | |
|---|---|---|---|---|
| | **Milieu 1** | | **Milieu 2** | |
| | % | N | % | N |
| **< 10** | 50,85 | 30 | 89,83 | 53 |
| **10 < Num <50** | 42,37 | 25 | 10,17 | 6 |
| **50 < Num <100** | 6,78 | 4 | 0,00 | 0 |
| **Tous** | 100,00 | 59 | 100,00 | 59 |

| | | | | |
|---|---|---|---|---|
| Avec NPP : nombre le plus probable | | | | |

On constate que parmi les 59 échantillons testés, le milieu 1 a conservé une bonne spécificité dans 50,85% des résultats de numération obtenus (NPP<10). 49,15% des résultats sont faussement positifs et liés à la croissance de *Bacillus* ont été obtenus dont 6.78% donnant des résultats de numération supérieurs à 50 UFC/g.

L'ajout de D-Alanine dans le milieu a permis d'améliorer le taux de résultats <10 de 50,85% à 89,83%. Le taux de résultats faux positifs a alors été réduit de 49,15% à 10,17%, et aucun résultat faux positif obtenu n'excède 50 UFC/gramme avec l'usage de D-Alanine.

## Revendications

1. Milieu de culture et de détection de staphylocoques à coagulase positive, comprenant :
• au moins un substrat spécifique, naturel ou synthétique, permettant la détection d'au moins une activité métabolique desdits staphylocoques à coagulase positive,dont le produit de dégradation fait varier le pH
• un indicateur de pH permettant de révéler la consommation dudit substrat
• au moins un composé inhibiteur ou retardateur de la germination de spores de *Bacillus,* susceptible d'interférer avec la culture et la détection desdits staphylocoques à coagulase positive ; ledit composé inhibiteur ou retardateur de la germination de spores étant choisi dans le groupe comprenant la D-alanine, la diphénylamine, les alcools de formule CₙH₂ₙ₊₂OH où n varie de 6 à 12, de préférence l'octanol, le nonanol, le décanol, les inhibiteurs d'enzymes de type trypsine et/ou leurs mélanges.

2. Milieu de culture selon la revendication 1, dans lequel la quantité du composé inhibiteur ou retardateur de la germination de spores, dans ledit milieu, est comprise entre 0,01 et 1 mol/L, préférentiellement 0,05 et 0,1 mol/L.

3. Milieu de culture selon l'une des revendications précédentes, **caractérisé en ce que** l'indicateur de pH permettant de révéler la consommation du substrat est un chromophore ou un fluorophore.

4. Milieu de culture selon l'une des revendications 1 à 3, dans lequel le substrat comprend une première partie spécifique de l'activité métabolique à révéler et une seconde partie faisant office de marqueur chromogène.

5. Milieu de culture selon l'une des revendications 1 à 3, dans lequel le substrat comprend une première partie spécifique de l'activité métabolique à révéler et une seconde partie faisant office de marqueur fluorescent.

6. Milieu de culture selon l'une des revendications précédentes, **caractérisé en ce que** les staphylocoques à coagulase positive sont des *Staphylococcus aureus.*

7. Procédé de détection de staphylocoques à coagulase positive susceptibles d'être présents dans un échantillon, comprenant les étapes consistant à :
a) Mettre en contact ledit échantillon avec un milieu de culture selon l'une des revendications 1 à 6,
b) Incuber ledit milieu de culture dans des conditions aptes à la multiplication cellulaire de microorganismes,
c) Détecter la présence desdits staphylocoques à coagulase positive.

8. Procédé selon la revendication 7, dans lequel l'étape c) consiste à mesurer la variation du niveau de fluorescence dans le milieu de culture à l'aide d'un lecteur de fluorescence, cette variation correspondent à un variation du pH dans ledit milieu de culture.

9. Procédé selon la revendications 7, dans lequel l'étape c) consiste à identifier les colonies.

10. Procédé selon la revendications 7 , dans lequel l'étape c) consiste à différencier les colonies de grandes tailles, des colonies de petites tailles.

11. Procédé selon l'une des revendications 7 à 10 dans lequel l'échantillon est un échantillon alimentaire ou environnemental.

12. Procédé selon l'une des revendications 7 à 11, comportant une étape supplémentaire de dénombrement de microorganismes cibles.

13. Utilisation d'au moins un composé retardant ou inhibant la germination de spores de microorganismes pour la préparation d'un milieu de culture selon l'une quelconque des revendications 1 à 6.

14. Utilisation de la D-alanine pour la préparation d'un milieu de culture selon l'une quelconque des revendications 1 à 6 destiné à l'identification des staphylocoques à coagulase positive tel que *Staphylococcus aureus.*

15. Utilisation d'un milieu de culture selon l'une des revendications 1 à 6, pour la détection, l'identifcation, voire le dénombrement des staphylocoques à coagulase positive tel que *Staphylococcus aureus.*

## Patentansprüche

1. Medium zur Kultur und zum Nachweis von Koagulase-positiven Staphylokokken, umfassend:
- mindestens ein natürliches oder synthetisches spezifisches Substrat, das den Nachweis mindestens einer Stoffwechselaktivität der Koagulase-positiven Staphylokokken ermöglicht, deren Abbauprodukt den pH-Wert verändert,
- einen pH-Indikator, der es ermöglicht, den Verbrauch des Substrats anzuzeigen,
- mindestens eine Verbindung, die die Bacillus-Sporenkeimung hemmt oder verzögert, die imstande ist, die Kultur und den Nachweis der Koagulase-positiven Staphylokokken zu stören;
wobei die Verbindung, die die Sporenkeimung hemmt oder verzögert, ausgewählt ist aus der Gruppe umfassend D-Alanin, Diphenylamin, Alkohole der Formel CₙH₂ₙ₊₂OH, wobei n zwischen 6 und 12 liegt, bevorzugt Octanol, Nonanol, Decanol, Hemmer der Enzyme vom Typ Trypsin und/oder deren Gemische.

2. Kulturmedium nach Anspruch 1, wobei die Menge der Verbindung, die die Sporenkeimung in dem Medium hemmt oder verzögert, im Bereich zwischen 0,01 und 1 mol/l, vorzugsweise 0,05 und 0,1 mol/l, liegt.

3. Kulturmedium nach einem der vorhergehenden Ansprüche, **dadurch gekennzeichnet, dass** der pH-Indikator, der es ermöglicht, den Verbrauch des Substrats anzuzeigen, ein Chromophor oder ein Fluorophor ist.

4. Kulturmedium nach einem der Ansprüche 1 bis 3, wobei das Substrat einen ersten Teil umfasst, der für die anzuzeigende Stoffwechselaktivität spezifisch ist, und einen zweiten Teil, der als Chromogen-Marker dient.

5. Kulturmedium nach einem der Ansprüche 1 bis 3, wobei das Substrat einen ersten Teil umfasst, der für die anzuzeigende Stoffwechselaktivität spezifisch ist, und einen zweiten Teil, der als Fluoreszenz-Marker dient.

6. Kulturmedium nach einem der vorhergehenden Ansprüche, **dadurch gekennzeichnet, dass** es sich bei den Koagulase-positiven Staphylokokken um *Staphylococcus aureus* handelt.

7. Verfahren zum Nachweis von Koagulase-positiven Staphylokokken, die imstande sind, in einer Probe vorhanden zu sein, das die folgenden Schritte umfasst:
a) Inkontaktbringen der Probe mit einem Kulturmedium nach einem der Ansprüche 1 bis 6,
b) Inkubieren des Kulturmediums unter Bedingungen, die für die Zellvermehrung von Mikroorganismen geeignet sind,
c) Nachweisen des Vorhandenseins der Koagulase-positiven Staphylokokken.

8. Verfahren nach Anspruch 7, wobei Schritt c) darin besteht, die Variation des Fluoreszenzniveaus in dem Kulturmedium mithilfe eines Fluoreszenzlesers zu messen, wobei diese Variation einer Variation des pH-Werts in dem Kulturmedium entspricht.

9. Verfahren nach Anspruch 7, wobei Schritt c) darin besteht, die Kolonien zu identifizieren.

10. Verfahren nach Anspruch 7, wobei Schritt c) darin besteht, die großen Kolonien von den kleinen Kolonien zu unterscheiden.

11. Verfahren nach einem der Ansprüche 7 bis 10, wobei es sich bei der Probe um eine Lebensmittel- oder eine Umweltprobe handelt.

12. Verfahren nach einem der Ansprüche 7 bis 11, das einen zusätzlichen Schritt zur Zählung von Ziel-Mikroorganismen umfasst.

13. Verwendung mindestens einer Verbindung, die die Sporenkeimung von Mikroorganismen verzögert oder hemmt, für die Herstellung eines Kulturmediums nach einem der Ansprüche 1 bis 6.

14. Verwendung von D-Alanin für die Herstellung eines Kulturmediums nach einem der Ansprüche 1 bis 6, das zur Identifikation von Koagulase-positiven Staphylokokken, wie beispielsweise *Staphylococcus aureus,* bestimmt ist.

15. Verwendung eines Kulturmediums nach einem der Ansprüche 1 bis 6 zum Nachweis, zur Identifikation oder sogar zur Zählung von Koagulase-positiven Staphylokokken, wie beispielsweise *Staphylococcus aureus.*

## Claims

1. A medium for the culture and detection of coagulase-positive staphylococci, comprising:
• at least one natural or synthetic specific substrate allowing the detection of at least one metabolic activity of said coagulase-positive staphylococci, the degradation product of which causes the pH to vary
• a pH indicator capable of revealing the consumption of said substrate
• at least one compound inhibiting or delaying the germination of Bacillus spores, which are capable of interfering with the culture and detection of said coagulase-positive staphylococci; said spore germination inhibiting or delaying compound is chosen from the group comprising D-alanine, diphenylamine, alcohols of formula CₙH₂ₙ₊₂OH where n ranges from 6 to 12, preferably octanol, nonanol or decanol, inhibitors of enzymes of trypsin type, and/or mixtures thereof.

2. The culture medium as claimed in claim 1, wherein the amount of the spore germination inhibiting or delaying compound in said medium is from 0.01 to 1 mol/l, preferably from 0.05 to 0.1 mol/l.

3. The culture medium as claimed in one of the preceding claims, wherein the pH indicator capable of revealing the substrate consumption is a chromophore or a fluorophore.

4. The culture medium as claimed in one of claims 1 to 3, wherein the substrate comprises a first part specific for metabolic activity to be revealed and a second part that acts as a chromogenic label.

5. The culture medium as claimed in one of claims 1 to 3, wherein the substrate comprises a first part specific for the metabolic activity to be revealed and a second part that acts as a fluorescent label.

6. The culture medium as claimed in one of the preceding claims, wherein the coagulase-positive staphylococci are *Staphylococcus aureus.*

7. A method for detecting coagulase-positive staphylococci that may be present in a sample, comprising the steps consisting in:
a) bringing said sample into contact with a culture medium as claimed in one of claims 1 to 6,
b) incubating said culture medium under conditions suitable for microorganism cell multiplication,
c) detecting the presence of said coagulase-positive staphylococci.

8. The method as claimed in claim 7, wherein step c) consists in measuring the variation in the level of fluorescence in the culture medium using a fluorescence reader, this variation corresponding to a variation in the pH in said culture medium.

9. The method as claimed in claim 7, wherein step c) consists in identifying the colonies.

10. The method as claimed in claim 7, wherein step c) consists in distinguishing the large colonies from the small colonies.

11. The method as claimed in one of claims 7 to 10, wherein the sample is a food or environmental sample.

12. The method as claimed in one of claims 7 to 11, comprising an additional step of counting target microorganisms.

13. The use of at least one compound delaying or inhibiting the germination of microorganism spores, for the preparation of a culture medium as claimed in any one of claims 1 to 6.

14. The use of D-alanine for the preparation of a culture medium as claimed in any one of claims 1 to 6 intended for identifying coagulase-positive staphylococci such as *Staphylococcus aureus.*

15. The use of a culture medium as claimed in one of claims 1 to 6, for the detection, identification or even counting of coagulase-positive staphylococci such as Staphylococcus aureus.
